# EUROPÄISCHE PATENTSCHRIFT

(11) **EP 1 302 158 B1**
(45) Veröffentlichungstag und Bekanntmachung des Hinweises auf die Patenterteilung: **17.05.2006**
(21) Anmeldenummer: 02090352.2
(22) Anmeldetag: 11.10.2002
(51) Int. Cl.: A61B 5/0464

(54) **Vorrichtung zur Vorhersage von Tachyarrythmien**
Device for predicting tachyarrythmias
Appareil de prédiction de tachyarythmie

(30) Priorität: 13.10.2001 DE 10151089
(43) Veröffentlichungstag der Anmeldung: 16.04.2003
(73) Patentinhaber: BIOTRONIK Mess- und Therapiegeräte GmbH & Co Ingenieurbüro Berlin, 12359 Berlin (DE)
(72) Erfinder: Fregien, Jörg, 15366 Hönow (DE); Dörr, Thomas, 12359 Berlin (DE); Hauser, Tino, 14163 Berlin (DE); Breithardt, Günter, 48149 Münster (DE); Fetsch, Thomas, 82131 Stockdorf (DE)
(74) Vertreter: Lindner-Vogt, Karin L.

(56) Entgegenhaltungen:
- EP-A- 0 469 817
- US-A- 4 880 005
- US-A- 5 749 900
- US-A- 6 115 627
- BRUYNE DE M C ET AL: "BOTH DECREASED AND INCREASED HEART RATE VARIABILITY ON THE STANDARD 10-SECOND ELECTROCARDIOGRAM PREDICT CARDIAC MORTALITY IN THE ELDERLY" AMERICAN JOURNAL OF EPIDEMIOLOGY, SCHOOL OF HYGIENE & PUBLIC HEALTH OF THE JOHNS, US, Bd. 150, Nr. 12, 15. Dezember 1999 (1999-12-15), Seiten 1282-1288, XP008020389 ISSN: 0002-9262

## Beschreibung

Die Erfindung betrifft eine Vorrichtung zur Vorhersage von Tachyarrythmien, welche Erfassungsmittel umfasst, die zur Aufnahme elektrischer Signale eines Herzens ausgebildet sind, sowie eine mit den Erfassungsmitteln verbundene Auswerteeinheit. Außerdem betrifft die Erfindung ein elektrisches Therapiegerät mit einer derartigen Vorrichtung. Das elektrische Therapiegerät ist dabei insbesondere ein Stimulator wie ein Herzschrittmacher, Kardioverter oder Defibrillator.

Entsprechende implantierbare Therapiegeräte sind grundsätzlich bekannt. Sie sind üblicherweise ausgebildet, elektrische Signale des Herzens zu erfassen und elektrische Impulse an ein Herz auszugeben. Eine entsprechende Erfassungseinheit für die elektrischen Signale ist dazu mit einer in einen Ventrikel und/oder ein Atrium des Herzens führenden Elektrodenleitung verbindbar. Eine solche Elektrodenleitung kann in bekannter Weise für die uni-, bi- oder multipolare Aufnahme elektrischer Signale aus dem Herzen und eine entsprechende Abgabe von therapeutischen Stromimpulsen an das Herz ausgebildet sein. Für die Aufnahme elektrischer Signale des Herzens sind beispielsweise bipolare Elektrodenleitungen geeignet, welche zusammen mit der Erfassungseinheit so geschaltet werden können, dass die erfassten Signale uni- oder bipolar aufgenommen werden. Für die Abgabe von Stimulations- oder Defibrillationsimpulsen an ein Herz umfasst ein derartiges Therapiegerät üblicherweise eine Stimulationseinheit. Erfassungs- und Stimulationseinheit eines solchen Implantates sind üblicherweise über eine Steuerung miteinander verbunden, die die Abgabe von Impulsen an das Herz in Abhängigkeit der vom Herzen aufgenommenen elektrischen Größen und gegebenenfalls weiterer Größen steuert. Solche weiteren Größen können im Falle eines ratenadaptiven Herzschrittmachers für den physiologischen Bedarf eines Patienten charakteristisch sein.

Zur Behandlung von Tachyarrythmien ist es insbesondere bekannt, eine Analyse der Herzratenvariabilität vorzunehmen. Gemäß des US-Patentes US 5,749,900 spricht eine entsprechende Vorrichtung auf einen Abfall der Variabilität der Herzrate an. Andere Vorrichtungen zur teilweise aufwendigen Analyse mehrerer Parameter sind beispielsweise aus der US 5,967,995 und der US 6,115,627 bekannt.

Eine entsprechende Vorhersage von Tachyarrythmien ist insbesondere für implantierbare Therapiegeräte von Interesse, um beispielsweise präventive oder prophylaktische Therapien rechtzeitig auslösen zu können.

Der vorliegenden Erfindung liegt die Aufgabe zu Grunde, eine Vorrichtung zur Vorhersage von Tachyarrythmien anzugeben, die die vorgenannten Anforderungen möglichst weitgehend erfüllt.

Erfindungsgemäß wird diese Aufgabe durch eine Vorrichtung der eingangs genannten Art gelöst, bei der die Auswerteeinheit zum Bestimmen eines von der jeweiligen Herzrate abhängigen Ratenwertes und zu einer Auswertung einer Mehrzahl entsprechend bestimmter Ratenwerte derart ausgebildet ist, dass die Auswertung einen Herzratenvariabilitätswert ergibt, der von der Variabilität der erfassten Ratenwerte abhängt, wobei die Auswerteeinheit auf einen Anstieg des Herzratenvariabilitätswertes anspricht.

Der Erfindung liegt die überraschende neue Erkenntnis zu Grunde, dass eine effektivere Vorhersage von Tachyarrythmien möglich ist, wenn eine entsprechende Auswerteeinheit abweichend vom Stand der Technik nicht auf einen Abfall der Herzratenvariabilität anspricht, sondern auf einen Anstieg.

Zusätzlich ist die Auswerteeinheit der eingangs genannten Vorrichtung ausgebildet, einen Abfall der Herzrate zu detektieren. Eine besonders sichere Detektion einer drohenden Tachyarrythmie bereits bis zu einer halben Stunde vor ihrem Auftreten lässt sich durch eine Kombination der Detektion eines Anstiegs der Herzratenvariabilität mit der gleichzeitigen Detektion des Absinkens der Herzfrequenz bzw. -rate erzielen.

Vorzugsweise ist die Auswerteeinheit ausgebildet, einen Anstieg der Herzratenvariabilität um 20%, besonders bevorzugt 30% zu detektieren. Zusätzlich ist sie ausgebildet, einen Abfall der Herzrate um 2%, vorzugsweise 5% zu detektieren.

In einer bevorzugten Ausführungsvariante ist die Auswerteeinheit ausgebildet, eine Standardabweichung der Ratenwerte als Herzratenvariabilitätswert zu bilden. Entsprechend spricht die Vorrichtung auf einen Anstieg dieser Standardabweichung an.

Um den Herzratenvariabilitätswert effektiv bilden zu können und eine Änderung der Herzrate effektiv zu ermitteln, umfasst eine bevorzugte Ausführungsvariante der Vorrichtung einen Ratenwertspeicher, der zum Speichern einer Mehrzahl aufeinander folgender Ratenwerte ausgebildet und zumindest mittelbar mit den Erfassungsmitteln und der Auswerteeinheit verbunden ist, wobei die Erfassungsmittel, die Auswerteeinheit und der Ratenwertspeicher derart zusammenwirken, dass eine jeweils aktuelle Folge von Ratenwerten in dem Ratenwertspeicher gespeichert ist und die Auswerteeinheit den Herzratenvariabilitätswert und die relative Änderung der Herzrate anhand der Ratenwerte in dem Ratenwertspeicher berechnet. Besonders bevorzugt ist es, wenn die Auswerteeinheit in diesem Falle ausgebildet ist, den Herzratenvariabilitätswert mit jedem neu erfassten Ratenwert zu bestimmen.

Eine bevorzugte Ausführungsvariante umfasst weiterhin eine Schwellwerteinheit, die mit der Auswerteeinheit verbunden oder in diese integriert und die ausgebildet ist, jeden Herzratenvariabilitätswert mit einem vorgegebenen Schwellwert zu vergleichen und bei Überschreiten des Schwellwertes ein Tachykardiewarnsignal zu erzeugen. Bei dieser Ausführungsvariante ist vorzugsweise ein Schwellwertspeicher vorgesehen, der zum Speichern des Schwellwertes ausgebildet und mit der Schwellwerteinheit verbunden ist. Dieser Schwellwertspeicher kann wiederbeschreibbar ausgeführt sein, so dass der Schwellwert an die jeweils aktuell vorliegende Situation durch einen Arzt angepasst werden kann.

Die Vorrichtung umfasst vorzugsweise weiterhin eine Tachykardieerfassungseinheit, die mit der Erfassungseinheit verbunden und ausgebildet ist, eine akute Tachykardie zu erfassen und ein Tachykardiesignal auszugeben.

Bei einer Vorrichtung, die sowohl einen Schwellwertspeicher als auch die vorgenannte Tachykardieerfassungseinheit aufweist, ist vorzugsweise weiterhin eine Schwellwertbestimmungseinheit vorgesehen, die mit dem Schwellwertspeicher und der Tachykardieerfassungseinheit verbunden und ausgebildet ist, den Schwellwert in dem Schwellwertspeicher zu verändern, falls die Tachykardieerfassungseinheit ein Tachykardiesignal ausgibt, ohne dass die Schwellwerteinheit zuvor ein der akuten Tachykardie zugeordnetes Tachykardiewarnsignal ausgegeben hat. Eine derartige Ausgestaltung der Vorrichtung ermöglicht in vorteilhafter Weise eine automatische Bildung eines geeigneten Schwellwertes dergestalt, dass vor einer akuten Tachykardie möglichst auch ein Tachykardiewarnsignal ausgegeben wird.

Weiterhin ist vorzugsweise eine Therapiesteuereinheit vorgesehen, die mit der Schwellwerteinheit bzw. der Auswerteeinheit verbunden und eine Stimulationseinheit umfasst, wobei die Therapiesteuereinheit so ausgebildet ist, dass sie eine prophylaktische Stimulation im Ventrikel mit einer Frequenz, die über der Grundfrequenz liegt, auslöst, wenn ein Tachykardiewarnsignal vorliegt. Umfasst die Vorrichtung auch eine Tachykardieerfassungseinheit, ist die Therapiesteuereinheit vorzugsweise mit dieser verbunden und so ausgebildet, dass sie eine antitachykarde Therapie oder eine Defibrillation auslöst, falls eine akute Tachykardie durch ein Tachykardiesignal der Tachykardieerfassungseinheit angezeigt wird.

Eine Vorrichtung der vorgenannten Art ist vorzugsweise Bestandteil eines elektrischen Therapiegerätes, insbesondere eines Stimulators wie beispielsweise eines Herzschrittmachers, Kardioverters oder Defibrillators.

Die Erfindung soll nun anhand eines Ausführungsbeispiels mit Hilfe der Figur näher erläutert werden.

### Die Figur zeigt einen implantierbaren Kardioverter/Defibrillator (ICD)

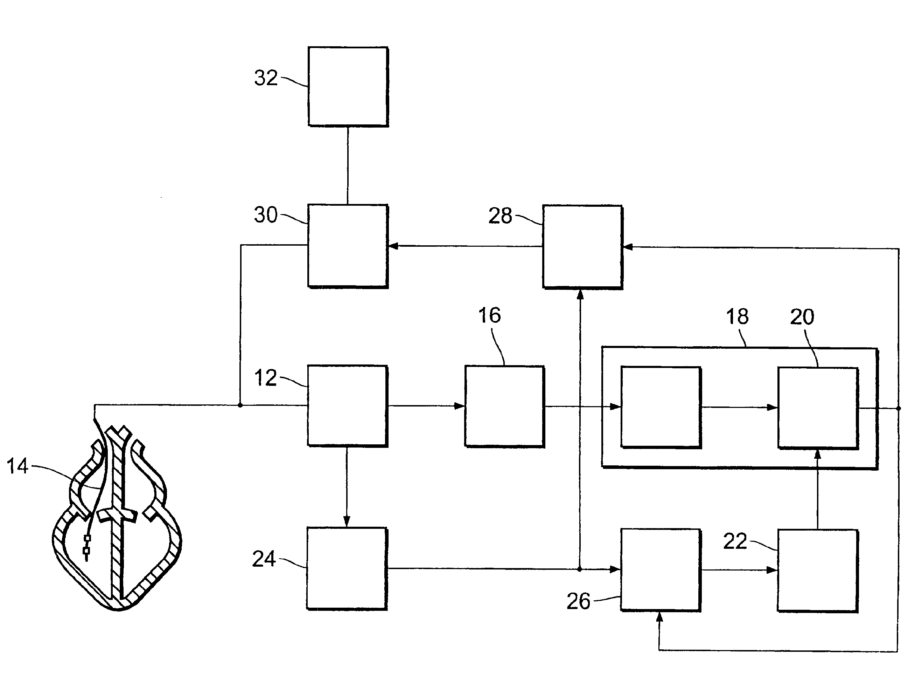

Das elektrische Therapiegerät aus Fig. 1 in Form eines implantierbaren Kardioverter/Defibrillators (ICD) 10 umfasst Erfassungsmittel 12 zum Erfassen elektrischer Signale eines Herzens über eine beispielsweise in den rechten Ventrikel eines Herzens reichende Elektrodenleitung 14. Die Erfassungsmittel 12 sind dementsprechend eingangsseitig mit der Elektrodenleitung 14 verbunden oder zumindest verbindbar. Die Erfassungsmittel 12 umfassen die üblichen Mittel zum Aufnehmen eines intrakardialen Elektrokardiogramms wie beispielsweise einen Eingangsverstärker, sowie einen QRS-Detektor, um den QRS-Komplex in dem intrakardial aufgenommenen Elektrokardiogramm zu detektieren. Weiterhin sind Zeitbestimmungsmittel vorgesehen, die mit dem QRS-Detektor so zusammenwirken, dass die Erfassungsmittel 12 die jeweils aktuelle Herzrate aus dem Abstand einer R-Zacke des QRS-Komplexes zur R-Zacke des nächstfolgenden QRS-Komplexes bestimmen können. Der auf diese Weise jeweils bestimmte aktuelle Herzratenwert wird in einen den Erfassungsmitteln 12 nachgeschalteten Ratenwertspeicher 16 ausgegeben, der ausgebildet ist, eine vorgegebene Anzahl der aktuellsten Herzratenwerte zu speichern.

Dem Ratenwertspeicher 16 ist eine Auswerteeinheit 18 nachgeschaltet, die eine Schwellwerteinheit 20 umfasst bzw. mit dieser verbunden ist. Die Auswerteeinheit 18 ist ausgebildet aus den in dem Ratenwertspeicher 16 gespeicherten, jeweils aktuellen Herzratenwerten eine Standardabweichung für diese Ratenwerte zu bilden. Ein dieser Standardabweichung entsprechender Wert wird der Schwellwerteinheit 20 zugeführt.

In der bevorzugten Ausführungsform ist die Auswerteeinheit zusätzlich ausgebildet, eine jeweils aktuelle, relative Änderung der Herzrate aus den in dem Ratenwertspeicher 16 gespeicherten, jeweils aktuellen Herzratenwerten zu ermitteln. Ein dieser relativen Herzratenänderung entsprechender Wert - z.B. eine Verringerung der Herzfrequenz um 5% - wird ebenfalls der Schwellwerteinheit 20 zugeführt. Zur Ermittlung der relativen Herzratenänderung wird z.B. ein aktueller Wert für die Herzrate mit einem über einen vorgegeben Zeitraum gemittelten Ratenwert verglichen und ins Verhältnis gesetzt. Der gemittelte Ratenwert ist wird ebenfalls mit jeder neuen Ratenerfassung angepasst.

Konkret sind beispielsweise die 5 oder 10 jeweils aktuellsten Herzratenwerte im Ratenwertspeicher 16 gespeichert. Anhand dieser Werte wird zum einen die Variabilität der Herzrate ermittelt, indem für diese Werte die Standardabweichung gebildet wird. Zum zweiten wird der aktuellste der Ratenwerte zum Mittelwert aller 5 oder 10 gespeicherten Ratentenwerte ins Verhältnis gesetzt, um die jeweils aktuelle, relative Ratenänderung zu bestimmen.

Die Schwellwerteinheit 20 ist mit einem Schwellwertspeicher 22 verbunden und ausgebildet, den jeweils aktuellen Wert der Standardabweichung und der relativen Herzratenänderung mit jeweils einem in dem Schwellwertspeicher 22 gespeicherten Wert zu vergleichen und ein Tachykardiewarnsignal auszugeben, falls die aktuelle Standardabweichung der Herzratenwerte den in dem Schwellwertspeicher 22 gespeicherten Referenzwert überschreitet und gleichzeitig oder zeitnah ein relativer Abfall der Herzfrequenz einen ebenfalls gespeicherten Schwellwert, entsprechend z.B. 3% Frequenzabfall, überschreitet.

Allein das Detektieren eines Anstiegs der Herzratenvariabilität über einen Schwellwert stellt einen entscheidenden Unterschied zur herrschenden Lehre dar, da die herrschende Lehre von einem Abfall der Herzratenvariabilität ausgeht. In vorteilhafter Weise wird gleichzeitig ein relativer Abfall der Herzfrequenz detektiert. Letzteres ist aber nicht unbedingt erforderlich.

Das Tachykardiewarnsignal wird einer Therapiesteuereinheit 28 zugeführt, die bei Anliegen des Tachykardiewarnsignals bewirkt, dass eine prophylaktische Stimulation im Ventrikel mit einer Frequenz, die über der Grundfrequenz liegt, ausgelöst wird. Dem dient eine Stimulationseinheit 30 mit einem Kondensator 32 als Energiespeicher, die ausgebildet, elektrische Impulse zur Therapie der drohenden Tachykardie über die Elektrodenleitung 14 an den Ventrikel eines Herzens abzugeben. Dementsprechend ist die Stimulationseinheit 30 ebenfalls mit der Elektrodenleitung 14 verbunden.

Um einen geeigneten Schwellwert für die Schwellwerteinheit 20 automatisch bilden und in dem Schwellwertspeicher 22 speichern zu können, ist der Schwellwertspeicher 22 eingangsseitig mit einer Schwellwertbestimmungseinheit 26 verbunden, die ihrerseits ausgebildet ist zwei Eingangssignale zu empfangen, nämlich ein Tachykardiesignal, welches von einer Tachykardieerfassungseinheit 24 im Falle einer akuten Tachykardie gebildet wird, sowie das Tachykardiewarnsignal, das von der Auswerteeinheit bzw. deren Schwellwerteinheit 20 generiert wird. Dazu ist die Tachykardieerfassungseinheit eingangsseitig mit den Erfassungsmitteln 12 verbunden. Als zweites Eingangssignal empfängt die Schwellwertbestimmungseinheit 26 das Tachykardiewarnsignal der Schwellwerteinheit 20. Liegt an der Schwellwertbestimmungseinheit 26 ein eine akute Tachykardie anzeigendes Tachykardiesignal der Tachykardieerfassungseinheit 24 an, ohne dass zuvor ein Tachykardiewarnsignal der Schwellwerteinheit 20 ausgegeben wurde, bewirkt die Schwellwertbestimmungseinheit ein Absenken des Schwellwertes im Schwellwertspeicher 22. Dies hat zum Ziel, dass die Schwellwerteinheit bereits auf geringere Anstiege der Standardabweichung der Herzratenwerte anspricht, so dass sie bereits früher ein Tachykardiewarnsignal ausgibt.

## Patentansprüche

1. Vorrichtung (10) zur Vorhersage von Tachyarrythmien, die Erfassungsmittel (12), welche zur Aufnahme elektrischer Signale eines Herzens ausgebildet sind, und eine mit den Erfassungsmitteln (12) verbundene Auswerteeinheit (18) umfasst, die Auswerteeinheit (18) ist zum Bestimmen eines von der jeweiligen Herzrate abhängenden Ratenwertes und zu einer Auswertung einer Mehrzahl entsprechend bestimmter Ratenwerte derart ausgebildet, dass die Auswertung einen Herzratenvariabilitätswert ergibt, der von der Variabilität der erfassten Ratenwerte abhängt, wobei die Auswerteeinheit (18) auf einen Anstieg des Herzratenvariabilitätswertes anspricht, **dadurch gekennzeichnet, dass** die Auswerteeinheit zusätzlich zur Detektion eines Abfalls der Herzrate ausgebildet ist.

2. Vorrichtung nach Anspruch 1, **dadurch gekennzeichnet, dass** die Auswerteeinheit (18) ausgebildet ist, eine Standardabweichung der Ratenwerte als Herzratenvariabilitätswert zu bilden.

3. Vorrichtung nach einem der Ansprüche 1 bis 2, **gekennzeichnet durch** einen Ratenwertspeicher (16), der zum Speichern einer Mehrzahl aufeinanderfolgender Ratenwerte ausgebildet und zumindest mittelbar mit den Erfassungsmitteln (12) und der Auswerteeinheit (18) verbunden ist, wobei die Erfassungsmittel (12), die Auswerteeinheit (18) und der Ratenwertspeicher (16) derart zusammenwirken, dass eine jeweils aktuelle Folge von Ratenwerten in dem Ratenwertspeicher gespeichert ist und die Auswerteeinheit (18) den Herzratenvariabilitätswert und den Abfall der Herzrate anhand der Ratenwerte in dem Ratenwertspeicher (16) berechnet.

4. Vorrichtung nach Anspruch 3, **dadurch gekennzeichnet, dass** die Auswerteeinheit (18) ausgebildet ist, den Herzratenvariabilitätswert und/oder den Abfall der Herzrate mit jedem neu erfassten Ratenwert zu bestimmen.

5. Vorrichtung nach einem der Ansprüche 1 bis 4, **gekennzeichnet durch** eine Schwellwerteinheit (20), die mit der Auswerteinheit (18) verbunden oder in diese integriert und die ausgebildet ist, jeden Herzratenvariabilitätswert und den Abfall der Herzrate mit einem jeweils vorgegebenen Schwellwert zu vergleichen und bei Überschreiten des Schwellwertes ein Tachykardiewarnsignal zu erzeugen.

6. Vorrichtung nach Anspruch 5, **dadurch gekennzeichnet, dass** die Schwellwerteinheit ausgebildet ist, einen Anstieg des Herzratenvariabilitätswertes um mehr als 20%, vorzugsweise mehr als 30% und einen Abfall der Herzrate um mehr als 2%, vorzugsweise mehr als 5% zu detektieren.

7. Vorrichtung nach Anspruch 5 oder 6, **gekennzeichnet durch** einen Schwellwertspeicher (22), der zum Speichern des Schwellwertes oder der Schwellwerte ausgebildet und mit der Schwellwerteinheit (20) verbunden ist.

8. Vorrichtung nach einem der Ansprüche 1 bis 7, **gekennzeichnet durch** eine Tachykardieerfassungseinheit (24), die mit der Erfassungseinheit (12) verbunden und ausgebildet ist eine akute Tachykardie zu erfassen und ein Tachykardiesignal auszugeben.

9. Vorrichtung nach Anspruch 7 und 8, **gekennzeichnet durch** eine Schwellwertbestimmungseinheit (26), die mit dem Schwellwertspeicher (22) und der Tachykardieerfassungseinheit (24) verbunden und ausgebildet ist, den Schwellwert oder die Schwellwerte in dem Schwellwertspeicher (22) zu verändern, falls die Tachykardieerfassungseinheit (24) ein Tachykardiesignal ausgibt, ohne dass die Schwellwerteinheit (20) zuvor ein der akuten Tachykardie zugeordnetes Tachykardiewarnsignal ausgegeben hat.

10. Vorrichtung nach Anspruch 6, **gekennzeichnet durch** eine Therapiesteuereinheit (28), die mit der Schwellwerteinheit (20) verbunden ist, sowie **durch** eine Stimulationseinheit (30) mit einem Energiespeicher (32), wobei die Therapiesteuereinheit (28) ausgebildet ist, eine prophylaktische Stimulation auszulösen, wenn ein Tachykardiewarnsignal anliegt.

11. Vorrichtung nach Anspruch 8 und 10, **dadurch gekennzeichnet, dass** die Therapiesteuereinheit (28) mit der Tachykardieerfassungseinheit (24) verbunden und ausgebildet ist, eine antitachykarde Therapie oder eine Defibrillation auszulösen, falls ein Tachykardiesignal anliegt.

12. Elektrisches Therapiegerät, insbesondere Stimulator, wie ein implantierbarer Herzschrittmacher, Kardioverter oder Defibrillator, **gekennzeichnet durch** eine Vorrichtung nach einem der Ansprüche 1 bis 10.

## Claims

1. A device (10) for predicting tachyarrhythmias, which comprises detection means (12), which are implemented to record electrical signals of a heart, and an analysis unit (18) connected to the detection means (12), the analysis unit (18) being implemented to determine a rate value as a function of the particular heart rate and to analyze multiple corresponding determined rate values in such a way that the analysis results in a heart rate variability value which is a function of the variability of the detected rate values, the analysis unit (18) responding to a rise of the heart rate variability value,
**characterized in that** the analysis unit is additionally implemented to detect a drop of the heart rate.

2. The device according to Claim 1,
**characterized in that** the analysis unit (18) is implemented to calculate a standard deviation of the rate values as the heart rate variability value.

3. The device according to one of Claim 1 through 2,
**characterized by** a rate value memory (16) which is implemented to store multiple sequential rate values and is at least indirectly connected to the detection means (12) and the analysis unit (18), the detection means (12), the analysis unit (18), and the rate value memory (16) working together in such a way that a particular current sequence of rate values is stored in the rate value memory and the analysis unit (18) calculates the heart rate variability value and the drop of the heart rate on the basis of the rate values in the rate value memory (16).

4. The device according to Claim 3,
**characterized in that** the analysis unit (18) is implemented to determine the heart rate variability value and/or the drop of the heart rate with each newly detected rate value.

5. The device according to one of Claims 1 through 4,
**characterized by** a threshold value unit (20), which is connected to the analysis unit (18) or integrated therein and which is implemented to compare each heart rate variability value and the drop of the heart rate to a particular predefined threshold value and to generate a tachycardia warning signal if the threshold value is exceeded.

6. The device according to Claim 5,
**characterized in that** the threshold value unit is implemented to detect a rise of the heart rate variability value by more than 20%, preferably more than 30%, and a drop of the heart rate by more than 2%, preferably more than 5%.

7. The device according to Claim 5 or 6,
**characterized by** a threshold value memory (22), which is implemented to store the threshold value or the threshold values and is connected to the threshold value unit (20).

8. The device according to one of Claims 1 through 7,
**characterized by** a tachycardia detection unit (24), which is connected to the detection unit (12) and is implemented to detect an acute tachycardia and output a tachycardia signal.

9. The device according to Claims 7 and 8,
**characterized by** a threshold value determination unit (26), which is connected to the threshold value memory (22) and the tachycardia detection unit (24) and is implemented to change the threshold value or the threshold values in the threshold value memory (22) if the tachycardia detection unit (24) outputs a tachycardia signal without the threshold value unit (20) previously having output a tachycardia warning signal assigned to acute tachycardia.

10. The device according to Claim 6,
**characterized by** a therapy control unit (28), which is connected to the threshold value unit (20), and by a stimulation unit (30) having a power accumulator (32), the therapy control unit (28) being implemented to trigger a prophylactic stimulation when a tachycardia warning signal is applied.

11. The device according to Claims 8 and 10,
**characterized in that** the therapy control unit (28) is connected to the tachycardia detection unit (24) and is implemented to trigger an anti-tachycardia therapy or a defibrillation if a tachycardia signal is applied.

12. An electrical therapy unit, particularly a stimulator, such as an implantable pacemaker, cardioverter, or defibrillator,
**characterized by** a device according to one of Claims 1 through 10.

## Revendications

1. Dispositif (10) destiné à prévoir des tachyarythmies, qui comprend des moyens de détection (12), qui sont réalisés pour l'enregistrement de signaux électriques d'un coeur, et une unité d'analyse (18) reliée aux moyens de détection (12), laquelle unité d'analyse (18) est réalisée pour le calcul d'une valeur de rythme dépendante du rythme cardiaque respectif et pour une analyse d'une pluralité de valeurs de rythme déterminées en conséquence de telle sorte que l'analyse donne une valeur de variabilité de rythme cardiaque qui dépend de la variabilité des valeurs de rythme détectées, l'unité d'analyse (18) réagissant à une élévation de la valeur de variabilité du rythme cardiaque, **caractérisé en ce que** l'unité d'analyse est conçue en supplément pour la détection d'une chute de rythme cardiaque.

2. Dispositif selon la revendication 1, **caractérisé en ce que** l'unité d'analyse (18) réagit pour former un écart-type des valeurs de rythme comme valeur de variabilité du rythme cardiaque.

3. Dispositif selon l'une quelconque des revendications 1 à 2, **caractérisé par** une mémoire de valeur de rythme (16), qui est conçue pour mémoriser une pluralité de valeurs de rythme successives et est reliée au moins indirectement aux moyens de détection (12) et à l'unité d'analyse (18), les moyens de détection (12), l'unité d'analyse (18) et la mémoire de valeur de rythme (16) coopérant de telle sorte qu'une succession respectivement actuelle de valeurs de rythme est mémorisée dans la mémoire de valeur de rythme et l'unité d'analyse (18) calcule la valeur de variabilité du rythme cardiaque et la baisse du rythme cardiaque à l'aide des valeurs de rythme dans la mémoire de valeur de rythme (16).

4. Dispositif selon la revendication 3, **caractérisé en ce que** l'unité d'analyse (18) est réalisée pour déterminer la valeur de variabilité du rythme cardiaque et/ou la baisse du rythme cardiaque avec toute nouvelle valeur de rythme enregistrée.

5. Dispositif selon l'une quelconque des revendications 1 à 4, **caractérisé par** une unité de valeur seuil (20), qui est reliée à l'unité d'analyse (18) ou est intégrée dans celle-ci et qui est réalisée pour comparer chaque valeur de variabilité de rythme cardiaque et la baisse du rythme cardiaque avec une valeur seuil respectivement prédéfinie et pour générer un signal d'alerte de tachycardie en cas de dépassement de la valeur seuil.

6. Dispositif selon la revendication 5, **caractérisé en ce que** l'unité de valeur seuil est réalisée pour détecter une élévation de la valeur de variabilité du rythme cardiaque de plus de 20 %, de préférence de plus de 30 % et une baisse du rythme cardiaque de plus de 2 %, de préférence de plus de 5 %.

7. Dispositif selon la revendication 5 ou 6, **caractérisé par** une mémoire de valeur seuil (22), qui est conçue pour mémoriser la valeur seuil ou les valeurs seuil et est reliée à l'unité de valeur seuil (20).

8. Dispositif selon l'une quelconque des revendications 1 à 7, **caractérisé par** une unité de détection de tachycardie (24), qui est reliée à l'unité de détection (12) et est réalisée pour détecter une tachycardie aiguë et émettre un signal de tachycardie.

9. Dispositif selon les revendications 7 et 8, **caractérisé par** une unité de calcul de valeur seuil (26), qui est reliée à la mémoire de valeur seuil (22) et à l'unité de détection de tachycardie et est conçue pour modifier la valeur seuil ou les valeurs seuils dans la mémoire de valeur seuil (22) dans le cas où l'unité de détection de tachycardie (24) envoie un signal de tachycardie sans que l'unité de valeur seuil (20) ait envoyé auparavant un signal d'alerte de tachycardie attribué à la tachycardie aiguë.

10. Dispositif selon la revendication 6, **caractérisé par** une unité de commande de thérapie (28), qui est reliée à l'unité de valeur seuil (20), ainsi que par une unité de stimulation (30) avec un accumulateur d'énergie (32), l'unité de commande de thérapie (28) étant conçue pour déclencher une stimulation prophylactique lorsqu'un signal d'alerte de tachycardie est appliqué.

11. Dispositif selon les revendications 8 et 10, **caractérisé en ce que** l'unité de commande de thérapie (28) est reliée à l'unité de détection de tachycardie (24) et est conçue pour déclencher une thérapie antitachycardique ou une défibrillation lorsqu'un signal de tachycardie est appliqué.

12. Appareil de thérapie électrique, en particulier un stimulateur, comme un pacemaker, un cardioverteur ou un défibrillateur greffable, **caractérisé par** un dispositif selon l'une quelconque des revendications 1 à 10.
